# FASCICULE DE BREVET EUROPEEN

(11) **EP 0 547 146 B1**
(45) Date de publication et mention de la délivrance du brevet: **19.07.1995**
(21) Numéro de dépôt: 91916722.1
(22) Date de dépôt: 02.09.1991
(51) Int. Cl.: A61B 17/22

(54) **CATHETER ET APPAREIL POUR LE TRAITEMENT NOTAMMENT DES EMBOLIES PULMONAIRES**
KATHETER UND VORRICHTUNG ZUR BEHANDLUNG, INSBESONDERE VON LUNGENEMBOLIEN
CATHETER AND APPARATUS FOR THE TREATMENT, INTER ALIA, OF PULMONARY EMBOLISMS

(30) Priorité: 04.09.1990 FR 9010982
(43) Date de publication de la demande: 23.06.1993
(73) Titulaire: Cannon, Robert Lee, III, F-72240 Conlie (FR)
(72) Inventeur: Cannon, Robert Lee, III, F-72240 Conlie (FR)
(74) Mandataire: David, Daniel
(86) Numéro de dépôt international: FR9100700
(87) Numéro de publication internationale: WO9203975

(56) Documents cités:
- FR-A- 1 394 733
- GB-A- 1 583 397
- US-A- 3 557 793
- US-A- 4 027 674
- US-A- 4 178 935

## Description

La présente invention a pour objet un cathéter permettant le traitement des embolies pulmonaires ou autres obstructions de vaisseaux sanguins, ainsi que l'appareil le comportant.

L'embolie pulmonaire massive, avec une obstruction de plus de 50 % de l'arbre artériel pulmonaire, reste une cause de mortalité importante malgré les progrès récents en thérapie médicale. Même si l'emploi de thrombolytiques est actuellement largement adopté comme premier traitement pour la majorité des patients souffrant de cette affection, il existe de nombreuses situations cliniques associées à l'embolie pulmonaire qui empêchent un tel traitement.

En outre, l'embolectomie chirurgicale pratiquée comme traitement de substitution présente un taux d'échecs relativement élevé et ne peut être envisagé que dans les hôpitaux disposant d'une installation pour la chirurgie cardiaque.

De même la thrombectomie transveineuse par cathéter, consistant à extraire le thrombus par entraînement au moyen d'une cupule mise en dépression, conduit à des complications fréquentes et sévères.

L'invention propose un nouveau moyen de traitement susceptible de se substituer aux techniques déjà connues, ne nécessitant pas la mise en oeuvre de moyens opératoires importants.

Conformément à l'invention, donc, on propose un cathéter pour la production d'ondes de choc conforme à la revendication 1.

Ce cathéter comprend notamment un corps tubulaire avec une extrémité proximale pour le raccordement à un appareil générateur d'impulsions de courant électrique, au moins une lumière longitudinale pour le logement de deux fils électriques isolés l'un de l'autre, et une extrémité distale de traitement avec deux électrodes reliées auxdits fils et disposées de façon à permettre la formation d'arcs électriques entre elles.

On utilise ce cathéter en l'introduisant, à l'aide d'un élément de guidage, dans un vaisseau sanguin conduisant jusqu'à la zone à traiter, et en positionnant l'extrémité distale à proximité
ou au contact du thrombus. On immobilise cette extrémité à l'intérieur du vaisseau sanguin, au moyen d'un ballonnet que l'on gonfle avec un fluide approprié. On connecte ensuite l'extrémité proximale à une source d'impulsions électriques appropriée de sorte que les décharges électriques produites entre les électrodes engendrent des ondes de choc qui se propagent à travers le liquide dans lequel baigne l'extrémité du cathéter. Ainsi, en choisissant convenablement le niveau d'énergie libéré par les impulsions de courant, la fréquence de ces derniers, et en amenant les électrodes suffisamment près de la thrombose, il est possible de recanaliser le vaisseau par simple effet mécanique. Les vibrations causées par la succession des ondes de choc liquides entraînent selon l'âge et la taille de la thrombose soit sa fluidification et son élimination, soit sa désagrégation en caillots plus petits susceptibles d'être traités plus facilement par les thrombolytiques. L'avantage de cette technique est en particulier de n'engendrer aucun risque de détérioration de la paroi du vaisseau sanguin du fait que l'arc électrique est parfaitement circonscrit entre les deux électrodes.

Elle permet en outre le traitement de thromboses qui sont résistantes aux traitements classiques.

D'autres avantages et caractéristiques apparaîtront à la lecture de la description qui suit de différents modes de réalisation non limitatifs de l'invention. Les dessins annexés représentent :
Figure 1 : un cathéter conforme à l'invention,
Figure 2 : une coupe transversale agrandie, selon II-II de la figure 1;
Figure 3 : une coupe transversale agrandie, selon III-III de la figure 1 ,
Figure 4 : une coupe longitudinale selon IV-IV de la figure 3,
Figure 5 : un schéma du circuit électrique de création des impulsions,
Figure 6 : un cathéter, vu en coupe longitudinale, selon un mode particulier de l'alimentation du ballonnet,
Figure 7 : un cathéter à extrémité atraumatique vue en coupe longitudinale,
Figures 8a et 8b : un cathéter, selon un deuxième mode de réalisation, vu respectivement en coupe longitudinale, selon VIIIa-VIIIa, et de face,
Figures 9a et 9b : un cathéter, selon un troisième mode de réalisation, vu respectivement en coupe longitudinale selon IXa-IXa, et de face.

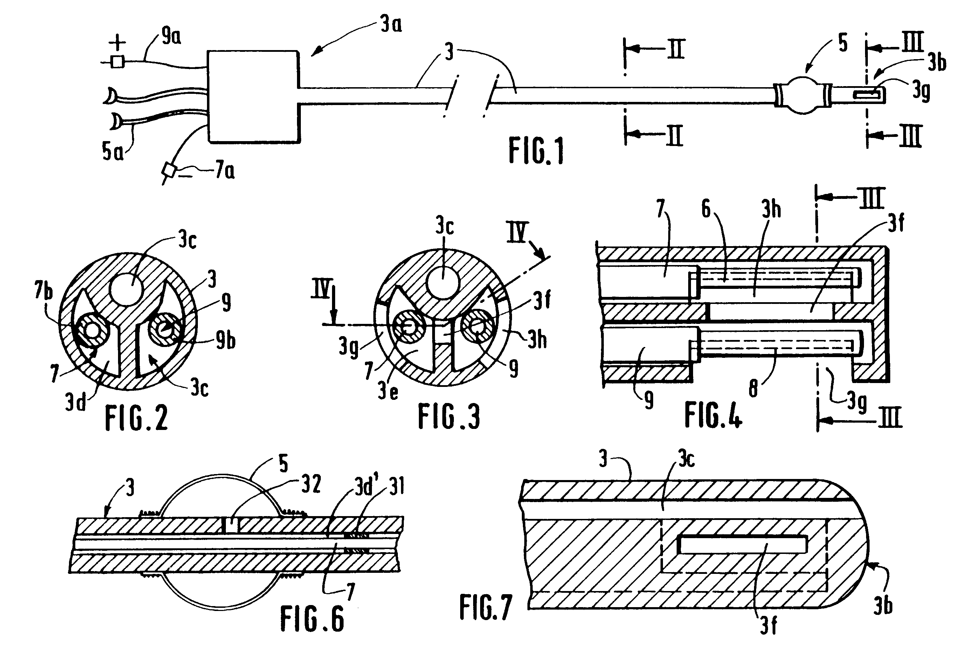

La figure 1 représente un cathéter conforme à l'invention repéré par la référence générale (1). Il est composé d'un corps tubulaire (3) réalisé en un matériau souple tel qu'un polychlorure de vinyle, un polytétrafluoréthylène, un polyéthyléne, etc. L'extrémité proximale (3a) du corps (3) comporte des moyens de raccordement à un appareil générateur électrique, et à diverses sources de fluides comme cela sera expliqué plus loin. L'extrémité distale (3b) comporte un moyen de production d'arcs électriques pour la création des ondes de choc par lesquels on parvient à désobstruer le vaisseau sanguin. Un moyen d'immobilisation (5) du corps tubulaire, à l'intérieur de la cavité du corps humain à traiter, est monté à proximité de l'extrémité distale du corps (3). Selon le mode de réalisation représenté, il est constitué par un ballonnet gonflable, connu en soi, relié, par une lumière longitudinale, ménagée à l'intérieur du corps (3), à une source de fluide sous pression approprié. La lumière se termine en (3a) par un élément de raccordement (5a), à ladite source de fluide. Le corps (3) est percé de deux lumières longitudinales (3d, 3e) pour le logement de deux éléments conducteurs de l'électricité (7,9) isolés, tels que des fils métalliques revêtus d'une gaine isolante (7b, 9b) comme cela est montré sur la figure 2. A leur extrémité proximale, les fils conducteurs sont pourvus d'éléments (7a, 9a) permettant leur raccordement au générateur électrique. Une lumière supplémentaire (3c) sert au passage d'un fil de guidage, connu en soi, pour amener le cathéter sur le site à traiter.

A proximité de l'extrémité distale du corps (3), les deux conducteurs sont reliés, chacun, à une électrode (6, 8) qui, dans l'exemple représenté sur les figures, sont obtenues par simple mise à nu des fils métalliques à leur extrémité distale. Les fils sont alors avantageusement en cuivre. Les ceux électrodes sont logées à l'intérieur du cathéter, dans les lumières (3d) et (3e). Celles-ci communiquent entre elles, au niveau des électrodes, par un perçage (3f) de la cloison qui les sépare. A ce niveau également, les lumières débouchent à l'extérieur par des fenêtres (3g, 3h) ménagées dans la paroi du corps tubulaire. L'extrémité du corps (3) est, dans cet exemple, obturée par un bouchon.

Le cathéter représenté est à 3 lumières, mais il est possible d'envisager d'autres solutions. Par exemple, si cela est nécessaire, on peut prévoir une lumière supplémentaire pour l'injection d'un fluide de contraste.

Selon un mode particulier de réalisation, la lumière d'alimentation du ballonnet en liquide physiologique, pour son gonflement, peut être avantageusement constituée par l'un des canaux (3d ou 3e) de guidage des conducteurs. Sur la figure 6, on a représenté en coupe longitudinale partielle une telle solution, au niveau du ballonnet. Le conducteur (7) est logé dans la lumière (3d) qui, près de son extrémité distale, est obturée par un bouchon (31). Une ouverture (32) ménagée dans la paroi du corps (3) communique avec le ballonnet monté de manière conventionnelle sur la sonde.

Sur la figure 7, on a représenté, vue en coupe longitudinale dans le plan de la cloison séparant les 2 lumières (3d, 3e), une extrémité (3b) de cathéter dont la forme est en calotte sphérique atraumatique afin de faciliter son déplacement le long des vaisseaux sanguins. En particulier, une telle forme est bien adaptée au traitement d'emboles récents dans lequel le cathéter peut pénétrer.

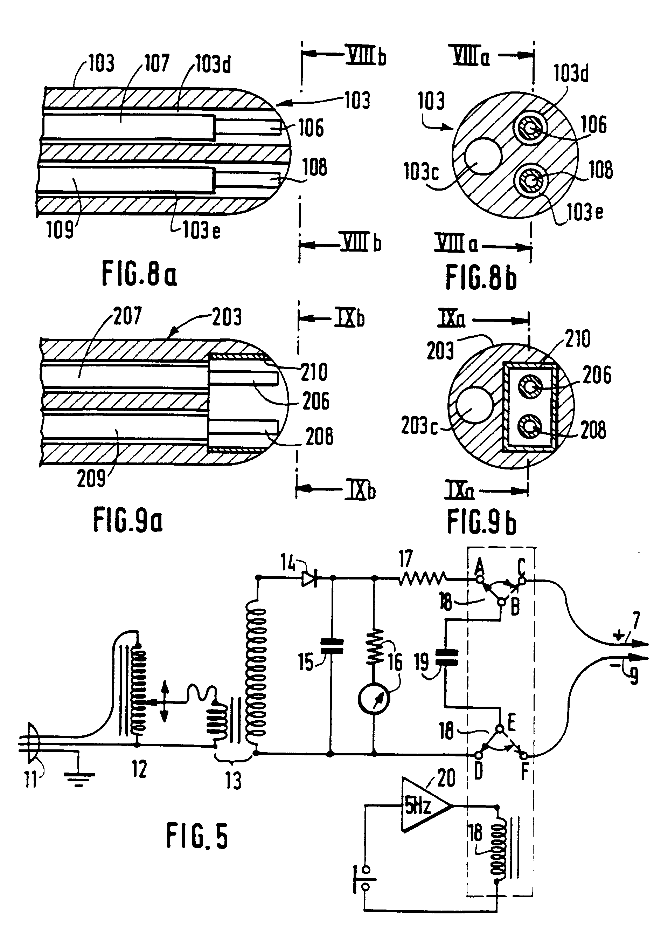

Sur les figures 8a et 8b, on a représenté un autre mode de réalisation dont les électrodes (106 et 108) reliées aux éléments conducteurs (107, 109) débouchent à l'extrémité de la sonde (103) de manière à produire des ondes de choc frontales. Dans ce mode de réalisation les lumières (103d, 103e) sont ouvertes à leur extrémité distale. Comme dans l'exemple de la figure 6, il est possible d'utiliser une des lumières (103d, 103e) de logement des conducteurs électriques (107, 109) pour alimenter le ballonnet que l'on n'a pas représenté, et qui est disposé de manière avantageuse près de l'extrémité de la sonde. La lumière (103c) sert au passage du fil de guidage. Selon une variante, représentée sur les figures 9a et 9b, les électrodes (206 et 208) sont logées à l'intérieur d'une bague (210) montée à l'extrémité du corps (203) et destinée à produire un effet directionnel sur les ondes de choc produites. L'extrémité des électrodes est de préférence en léger retrait par rapport à la surface frontale, elle-même de préférence de forme atraumatique, de la sonde (203).

A son extrémité proximale, le cathéter est raccordé à un générateur d'impulsions électriques dont un exemple de schéma est montré à la figure 5.

Sur le schéma, le transformateur élévateur de tension (13) est relié à une source d'énergie (11) par l'intermédiaire d'un auto-transformateur variable (12). Le transformateur alimente un redresseur de courant (14) qui convertit le courant alternatif en courant continu et charge le condensateur (15). Un appareil de mesure (16) est branché entre les bornes du condensateur (15). Un deuxième condensateur (19) est branché également entre les bornes (A, D) du condensateur (15) par l'intermédiaire d'un double relais (18). Dans une première position le relais (18) met en communication les bornes respectives des condensateurs (15) et (19) de façon à à permettre le chargement du deuxième (19) par le premier (15). On prévoit une résistance (17) entre les deux condensateurs pour limiter l'intensité du courant de charge. Dans une deuxième position, le relais (18) met en communication les bornes (B, E) du condensateur (19) avec deux plots (C, F) qui sont reliés chacun respectivement aux fils (7, 9) du cathéter.

Le circuit de commande du relais (18) comprend un oscillateur (20) dont la fréquence détermine celle des décharges électriques produisant les ondes de choc souhaitées. En effet à chaque mise en communication des bornes (B, E) avec les plots (C, F), le relais permet le déchargement du condensateur (19) dans le circuit comprenant les deux électrodes (6, 8) et d'engendrer un arc électrique entre elles.

On peut ainsi de façon très simple contrôler la puissance, la durée et la fréquence des chocs en choisissant convenablement la tension du transformateur, la capacité des condensateurs et la fréquence de l'oscillateur.

L'énergie libérée par les décharges électriques sera comprise entre 1 et 1000 mJ, et de préférence entre 1 et 100 mJ, et la fréquence des impulsions comprise entre 1 et 10 Hertz. Des essais ont été effectués où une énergie de 20 mJ s'est révélée suffisante pour assurer la désobstruction d'un vaisseau sanguin.

Ce montage présente en outre l'avantage d'isoler le cathéter du secteur d'alimentation au moment de la décharge, et offre ainsi une grande sécurité d'utilisation.

## Revendications

1. Cathéter pour la production d'ondes de choc comprenant un corps tubulaire (3, 103, 203) avec une extrémité proximale (3a) pour le raccordement à un appareil générateur d'impulsions de courant électrique, au moins une lumière longitudinale (3d, 3e; 103d, 103e ; 203d, 203e) dans laquelle sont logés deux éléments (7, 9 ; 107, 109 ; 207, 209) conducteurs de l'électricité et une extrémité distale (3b) de traitement avec deux électrodes (6, 8 ; 106, 108 ; 206, 208), reliées auxdits fils, pour la production d'arcs électriques générateurs des ondes de choc caractérisé en ce qu' il est destiné au traitement des embolies pulmonaires ou autres obstructions de vaisseaux sanguins et en ce qu'il comporte à son extrémité distale un moyen (5) d'immobilisation dudit corps tubulaire à l'intérieur du vaisseau sanguin obstrué, ledit corps tubulaire comportant une lumière permettant le passage d'un fil de guidage.

2. Cathéter selon revendication précédente caractérisé en ce que les conducteurs (7, 9 ; 107, 109 ; 207, 209) sont des fils métalliques revêtus d'une gaine isolante et les électrodes (6, 8 ; 106, 108 ; 206, 208) sont obtenues par mise à nu desdits fils métalliques à leur extrémité distale.

3. Cathéter selon la revendication précédente caractérisé en ce que ledit moyen d'immobilisation est constitué par un ballonnet (5) monté à l'extrémité du corps (3), et communiquant par une lumière ménagée dans le corps tubulaire avec un moyen d'alimentation en fluide sous pression.

4. Cathéter selon la revendication 3 caractérisé en ce que le moyen d'alimentation en fluide sous pression est constitué par l'une des lumières longitudinales (3d) pour le logement des éléments conducteurs.

5. Cathéter selon l'une des revendications 1 à 4 caractérisé en ce que les électrodes (6, 8) sont logées à l'intérieur du corps tubulaire (3), et sont espacées l'une de l'autre sur au moins une portion de leur longueur de façon à pouvoir produire entre elles un arc électrique à travers une ouverture (3f) ménagée entre les deux lumières (3d, 3e) l'onde de choc se propageant à travers des fenêtres (3g, 3h) ménagées dans la paroi dudit corps tubulaire (3).

6. Cathéter selon l'une des revendications 1 à 4 caractérisé en ce que les électrodes (106 et 108) débouchent à l'extrémité du corps tubulaire (103) pour produire des ondes de choc frontales.

7. Cathéter selon la revendication 6 caractérisé en ce que les électrodes sont logées à l'intérieur d'une bague (210) destinée à produire un effet directionnel sur les ondes de choc.

8. Cathéter selon l'une des revendications précédentes caractérisé en ce qu'il est raccordé à un appareil de production de décharges électriques, susceptibles de créer des arcs électriques sous forme d'impulsions à une fréquence comprise entre 1 et 10 Hertz.

9. Cathéter selon la revendication précédente caractérisé en ce que ledit appareil produit des décharges électriques d'énergie comprise entre 1 et 1000 mJ.

10. Cathéter selon la revendication précédente caractérisé en ce que ledit appareil produit des décharges électriques d'énergie comprise entre 1 et 100 mJ.

11. Matériel pour le traitement notamment d'embolies pulmonaires caractérisé en ce qu'il est composé d'un cathéter conforme à l'une des revendications 1 à 9 et d'un appareil de production de décharge électriques, susceptible de créer des arcs électriques, sous forme d'impulsion à une fréquence comprise entre 1 et 10 Hertz.

12. Matériel selon la revendication précédente caractérisé en ce que ledit appareil est susceptible de produire des décharges électriques d'énergie comprise entre 1 et 1000 mJ.

## Claims

1. Catheter for the production of shock waves comprising a tubular body (3, 103, 203) with a proximal end (3a) for connection to apparatus generating electric current pulses, at least one longitudinal hole (3d, 3e; 103d, 103e; 203d, 203e) in which are housed two electrically conductive elements (7, 9; 107, 109; 207, 209), and a distal treatment end (3b) with two electrodes (6, 8; 106, 108; 206, 208) connected to the said wires, for producing electric arcs generating shock waves, characterised in that it is designed for the treatment of pulmonary embolisms or other blood vessel obstructions, and in that it has at its distal end a means (5) for immobilizing the said tubular body inside the obstructed blood vessel, the said tubular body having a hole allowing the passage of a guide wire.

2. Catheter according to the preceding claim, characterised in that the conductors (7, 9; 107, 109; 207, 209) are metal wires covered with an insulating sheath and the electrodes (6, 8; 106, 108; 206, 208) are obtained by baring the said metal wires at their distal end.

3. Catheter according to the preceding claim, characterised in that the said immobilization means consists of a small balloon (5), mounted at the end of the body (3), and communicating through a hole formed in the tubular body with a pressurized fluid supply means.

4. Catheter according to Claim 3, characterised in that the pressurized fluid supply means consists of one of the longitudinal holes (3d) for housing the conductor elements.

5. Catheter according to one of Claims 1 to 4, characterised in that the electrodes (6, 8) are housed inside the tubular body (3) and are spaced apart from each other over at least part of their length so as to be able to produce between them an electric arc through an opening (3f) formed between the two holes (3d, 3e), the shock wave propagating through the windows (3g, 3h) formed in the wall of the said tubular body (3).

6. Catheter according to one of Claims 1 to 4, characterised in that the electrodes (106 and 108) open out at the end of the tubular body (103) in order to produce frontal shock waves.

7. Catheter according to Claim 6, characterised in that the electrodes are housed inside a ring (210) designed to produce a directional effect on the shock waves.

8. Catheter according to one of the preceding claims, characterised in that it is connected to apparatus for the production of electrical discharges able to create electric arcs in the form of pulses at a frequency between 1 and 10 hertz.

9. Catheter according to the preceding claim, characterised in that the said apparatus produces electrical discharges whose energy is between 1 and 1000 mJ.

10. Catheter according to the preceding claim, characterised in that the said apparatus produces electrical discharges whose energy is between 1 and 100 mJ.

11. Equipment for the treatment, notably, of pulmonary embolisms, characterised in that it consists of a catheter in accordance with one of Claims 1 to 9 and apparatus for producing electrical discharges, able to create electric arcs in the form of pulses at a frequency between 1 and 10 hertz.

12. Equipment according to the preceding claim, characterised in that the said apparatus is able to produce electrical discharges with an energy between 1 and 1000 mJ.

## Patentansprüche

1. Katheter zum Herstellen von Stoßwellen mit einem röhrenförmigen Körper (3, 103, 203) mit einem proximalen Ende (3a) für die Verbindung mit einer Vorrichtung zum Erzeugen von elektrischen Stromimpulsen, zumindest einem longitudialen Loch (3d, 3e; 103d, 103e; 203d, 203e), in dem zumindest zwei elektrische Leitungselemente (7, 9; 107, 109; 207, 209) untergebracht sind und einem distalen Ende (3d) zur Behandlung mit zwei Elektroden (6, 8; 106, 108; 206, 208), die mit den Drähten verbunden sind, zur Herstellung von elektrischen Lichtbögen zur Erzeugung von Stoßwellen, dadurch gekennzeichnet, daß er für die Behandlung von Lungenembolien oder anderen Verschlüssen von Blutgefäßen bestimmt ist und das er an seinem distalen Ende ein Mittel (5) zum Immobilisieren des röhrenförmigen Körpers im Innern des verstopften Blutgefäßes aufweist, wobei der röhrenförmige Körper ein Loch aufweist, das den Durchgang eines Führungsdrahtes ermöglicht.

2. Katheter gemäß vorstehendem Anspruch, dadurch gekennzeichnet, daß die Leiter (7, 9; 107, 109; 207, 209) mit einer isolierenden Umhüllung bedeckte metallische Drähte sind und die Elektroden (6, 8; 106, 108; 206, 208) durch Freilegen der metallischen Drähte an ihrem distalen Ende erhalten sind.

3. Katheter gemäß dem vorstehenden Anspruch, dadurch gekennzeichnet, daß das Mittel zum Immobilisieren von einem Ballonett (5) gebildet ist, welches an dem Ende des Körpers (3) angebracht ist und über ein in den röhrenförmigen Körper eingearbeitetes Loch mit einem Mittel zum Einspeisen von Fluid unter Druck verbunden ist.

4. Katheter gemäß Anspruch 3, dadurch gekennzeichnet, daß das Mittel zum Einspeisen von Fluid unter Druck von dem einen der longitudinalen Löcher (3d) zum Unterbringen der leitenden Elemente gebildet ist.

5. Katheter gemäß einem der Ansprüche 1 bis 4, dadurch gekennzeichnet, daß die Elektroden (6, 8) im Innern des röhrenförmigen Körpers (3) untergebracht und voneinander auf zumindest einem Teil ihrer Länge auf eine Weise beabstandet sind, daß sie zwischen sich einen elektrischen Lichtbogen über eine Öffnung (3f) erzeugen können, die zwischen den beiden Löchern (3d, 3e) eingearbeitet ist, wobei sich die Stoßwelle durch Fenster (3g, 3h) ausbreitet, die in die Wand röhrenförmigen Körpers (3) eingearbeitet sind.

6. Katheter gemäß einem der Ansprüche 1 bis 5, dadurch gekennzeichnet, daß die Elektroden (106 und 108) am Ende des röhrenförmigen Körpers (103) münden, um frontale Stoßwellen zu erzeugen.

7. Katheter gemäß Anspruch 6, dadurch gekennzeichnet, daß die Elektroden im Inneren eines Ringes (210) angeordnet sind, der bestimmt ist, eine richtende Wirkung auf die Stoßwellen auszuüben.

8. Katheter gemäß einem der vorstehenden Ansprüche, dadurch gekennzeichnet, daß er mit einer Vorrichtung zum Erzeugen von elektrischen Entladungen verbunden ist, die geeignet sind, elektrische Lichtbögen in Form von Impulsen zu erzeugen, mit einer Frequenz, die zwischen 1 und 10 Hertz enthalten ist.

9. Katheter gemäß dem vorstehenden Anspruch, dadurch gekennzeichnet, daß die Vorrichtung elektrische Entladungen von einer Energie enthalten zwischen 1 und 1000 mJ erzeugt.

10. Katheter gemäß dem vorstehendem Anspruch, dadurch gekennzeichnet, daß die Vorrichtung elektrische Entladungen mit einer Energie enthalten zwischen 1 und 100 mJ erzeugt.

11. Gerätschaft zur Behandlung insbesondere von Lungenembolien, dadurch gekennzeichnet, daß es ein Katheter gemäß einem der Ansprüche 1 bis 9 und eine Vorrichtung zum Erzeugen von elektrischen Entladungen, die geeignet zum Erzeugen elektrischer Lichtbögen sind, in Form von Impulsen mit einer Frequenz enthalten zwischen 1 und 10 Hertz aufweist.

12. Gerätschaft gemäß vorstehendem Anspruch, dadurch gekennzeichnet, daß die Vorrichtung geeignet ist, elektrische Entladungen einer Energie enthalten zwischen 1 und 1000 mJ zu erzeugen.
